# EUROPEAN PATENT APPLICATION

(11) **EP 3 623 813 A1**
(43) Date of publication of application: **18.03.2020**
(21) Application number: 18382669.2
(22) Date of filing: 17.09.2018
(51) Int. Cl.: G01N 33/569

(54) **METHODS FOR THE PROGNOSIS OF HIV-INFECTED SUBJECTS**

(71) Applicant: Institut d'Investigació Sanitària Pere Virgili, 43003 Tarragona (ES); Universitat Rovira i Virgili, 43003 Tarragona (ES); Servicio Andaluz de Salud, E-41071 Sevilla (ES)
(72) Inventor: RODRÍGUEZ GALLEGO, Esther, 43005 Tarragona (ES); RULL AIXA, Anna, 43005 Tarragona (ES); VIDAL MARSAL, Francesc, 43005 Tarragona (ES); ALBA ELVIRA, Verónica, 43005 Tarragona (ES); RUIZ-MATEOS CARMONA, Ezequiel, 41013 Sevilla (ES); LÓPEZ CORTÉS, Luis Fernando, 41013 Sevilla (ES); TARANCÓN DÍEZ, Laura, 41013 Sevilla (ES); PERAIRE FORNER, Joaquim, 43005 Tarragona (ES); VILADÉS LABORDA, Consuelo, 43005 Tarragona (ES)
(74) Representative: ABG Intellectual Property Law, S.L.

(57) **Abstract**

The invention provides an *in vitro* method, kits, uses of said kits and of reagents, for determining the prognosis of an HIV-infected subject having an undetectable viral load in the absence of antiretroviral treatment (or of an Ellite Controler HIV-infected subject) by determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a sample from said subject, and comparing the expression level of said at least one biomarker with a reference value.

## Description

### FIELD OF THE INVENTION

The invention relates to the field of prognosis in a subset of HIV-infected subjects, more specifically, to methods for the prognosis of HIV-infected subjects showing undetectable viral load in the absence of antiretroviral treatment, particularly subjects that can be classified as Elite Controllers (EC), based on the expression level of a gene or a set of genes.

### BACKGROUND ART

The Human Immunodeficiency Virus (HIV) infection is characterized either by an increased plasmatic viremia or by a progressive loss of CD4+ T lymphocytes. In the absence of antiretroviral therapy, this results in an important immunologic impairment with the consequent appearance of opportunistic infections and, lastly, death.

However, the so-called Elite Controllers (EC), who represent a minority group of subjects in the scenario of human immunodeficiency virus type 1 (HIV-1) infection (ca. 1%), are able to maintain an undetectable viral load (VL) in the absence of combined antiretroviral treatment (Lambotte O. and Delfraissy J-F., Pathol. Biol. 2006; 54:566-571). Due to this characteristic, EC have been proposed as a model of functional cure, eradication strategies and also HIV vaccine development. Knowledge of the mechanisms involved in the controller phenomenon is highly relevant for the identification of the virological and host determinants involved in the spontaneous control.

Host genetic factors, mainly HLA class I molecules, such as HLA-B*57, as well as immunological mechanisms, such as the HIV-1-specific T-cell response characterized by increased production of cytokines, chemokines and cytolytic enzymes (named polyfunctionality), and HIV-1-suppression capacity have been associated with this clinical situation.

New evidence suggests that these subjects have heterogeneous clinical outcomes including a variable proportion, around 25%, who loses HIV control overtime (Noel N. et al., PLoS One 2015; 10:1-11; Leon A. et al., AIDS 2016; 30:1209-1220). Said proportion of EC subjects are called Transient Controllers (TC), whereas those maintaining control on viral replication over time are considered Persistent Controllers (PC). To date, no longitudinal study elucidating the proteomic profile associated with the loss of spontaneous HIV-1 elite control has been performed. Thus, information allowing understanding the development of the condition, as well as allowing the early identification of TC subjects is missing. However, understanding the mechanisms involved in the development of the condition is needed, to allow identifying the most accurate therapy for said subjects, and to allow an early identification of TCs before the loss of viral control occurs thus increasing the probabilities of recovery, or of maintaining the viral replication control.

Thus, despite the advances made describing the characteristics of TC subjects, there is a need for methods allowing the identification of TC subjects before the loss of viral control is detected.

### SUMMARY OF THE INVENTION

In a first aspect, the invention relates to an *in vitro* method for determining the prognosis or progression of an HIV-infected subject having an undetectable viral load in the absence of antiretroviral treatment, which comprises:
(i) determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a sample from said subject, and
(ii) comparing the expression level of said at least one biomarker with a reference value,
wherein if the expression level of at least one biomarker selected from LG3BP, AACT, FCN2 and 1433Z is increased with respect to the reference value, and/or the level of expression of FA11 is decreased with respect to the reference value, then the subject shows bad prognosis, or
wherein if the expression level of at least one biomarker selected from LG3BP, AACT, FCN2 and 1433Z is decreased or lacks change with respect to the reference value, and/or the level of expression of FA11 is increased or lacks change with respect to the reference value, then the subject shows good prognosis.

In a second aspect, the invention relates to a kit comprising reagents adequate for the determination of the expression level of at least one biomarker selected from the group consisting of LG3BP, AACT, FCN2, 1433Z and FA11.

In a third aspect, the invention relates to the use of a kit according to the second aspect or of a reagent capable of determining the expression level of at least one biomarker selected from the group consisting of LG3BP, AACT, FCN2, 1433Z and FA11 for determining the prognosis or progression of an HIV-infected subject having anundetectable viral load in the absence of antiretroviral treatment.

### BRIEF DESCRIPTION OF THE DRAWINGS

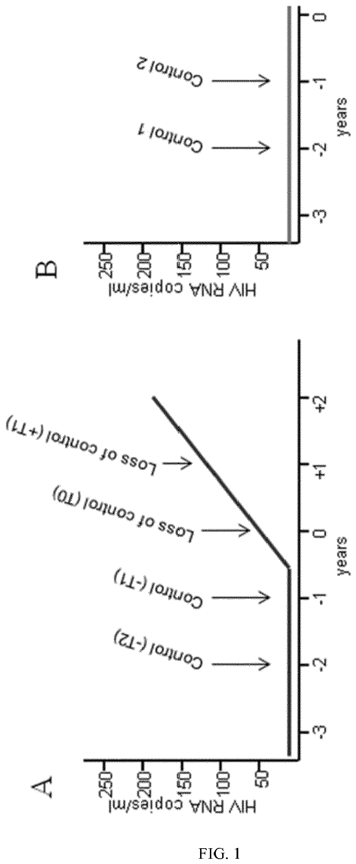

**Figure 1** shows a study design. A schematic representation of the study design in Transient Controllers (TC) (A) and Persistent Controllers (PC) (B).

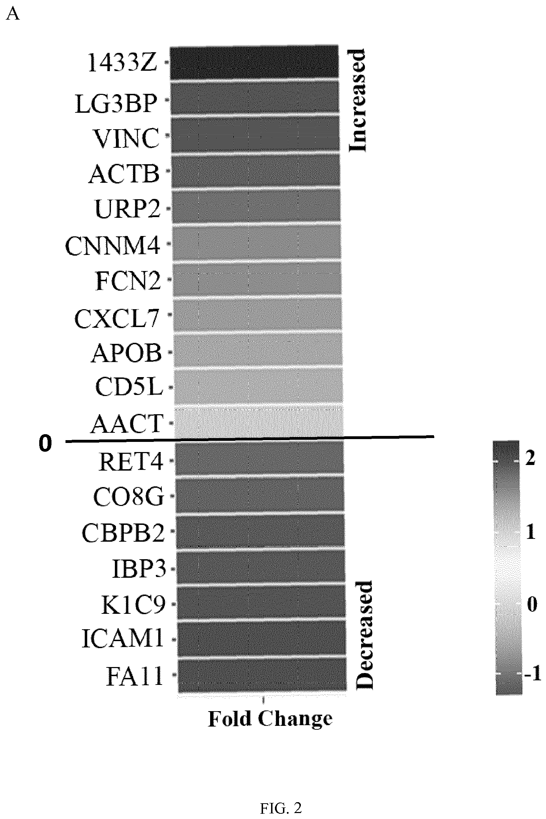

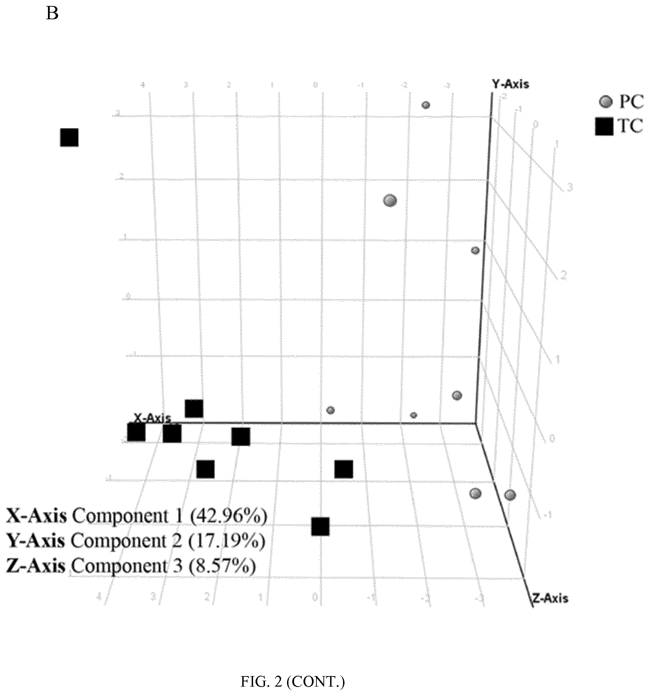

**Figure 2** shows a proteomic analysis comparing TC before the loss of natural HIV-1 control and PC, and a heatmap representation of quantified statistically significant proteins (A). PCA showing that these proteins allow differentiation between the studied groups (B), TC (squares, n=8) and PC (circles, n=8). The scale from light grey (zero fold change) to dark grey (high fold change in absolute values) represents the normalized abundance in arbitrary units. Increased fold changes refer to positive fold changes, whereas decreased fold changes refer to negative fold changes.

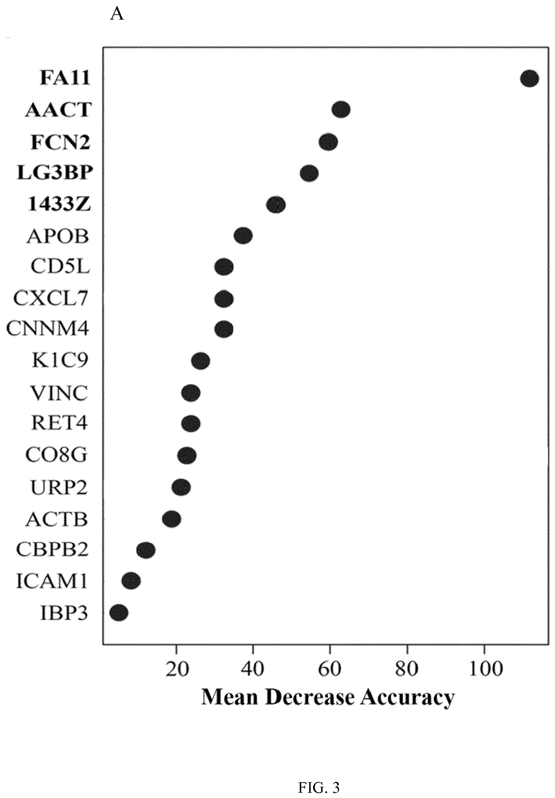

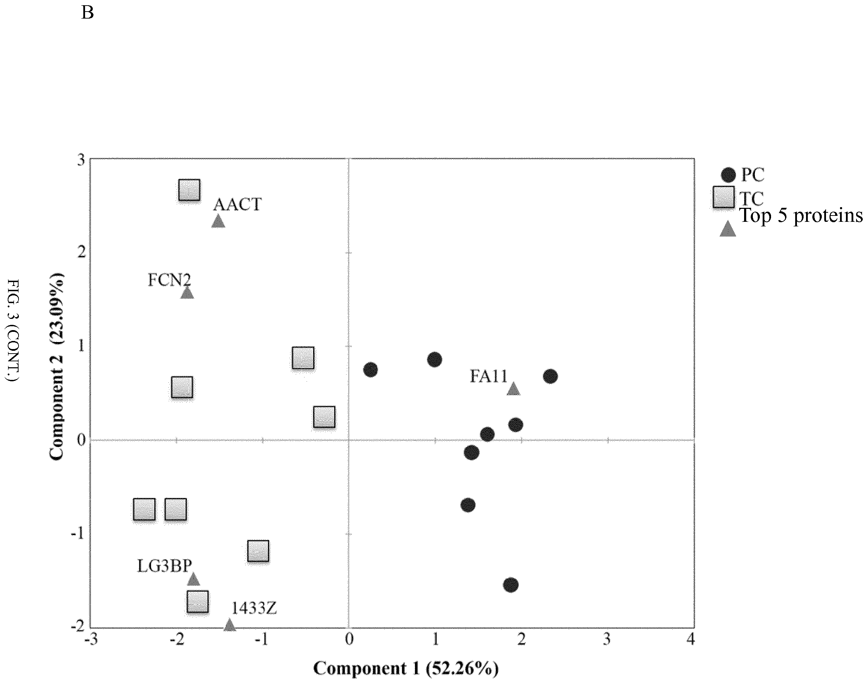

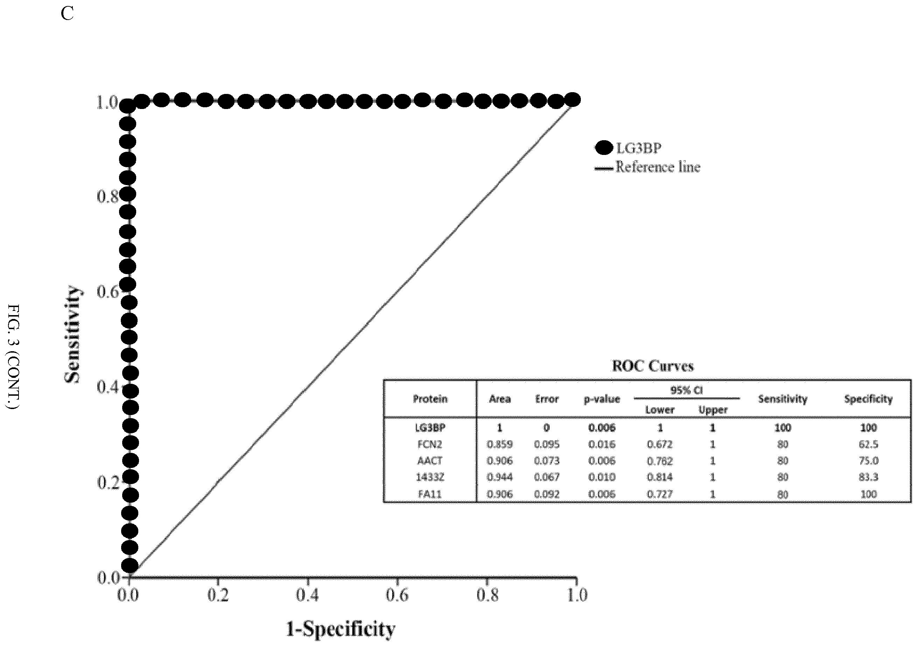

**Figure 3** shows proteins that are potential biomarkers of the loss of virological control. Random Forest analysis plot of the 18 proteins ordered by importance of classification. Only the top five were considered potential biomarkers (A). Score plot of the PCA using this top five entities (triangles) showed good differentiation between TC (squares, n=8) and PC (circles, n=8) (B). Logistic regression and receiver operator characteristic (ROC) curves elucidated Galectin-3-binding protein (LG3BP) as the most reliable biomarker for the prediction of the spontaneous loss of virological control in EC. (C).

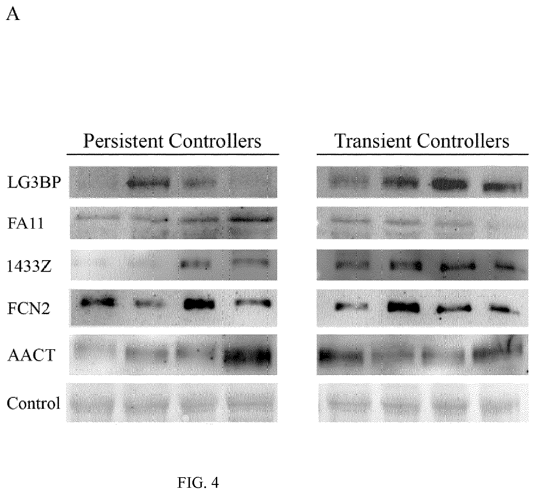

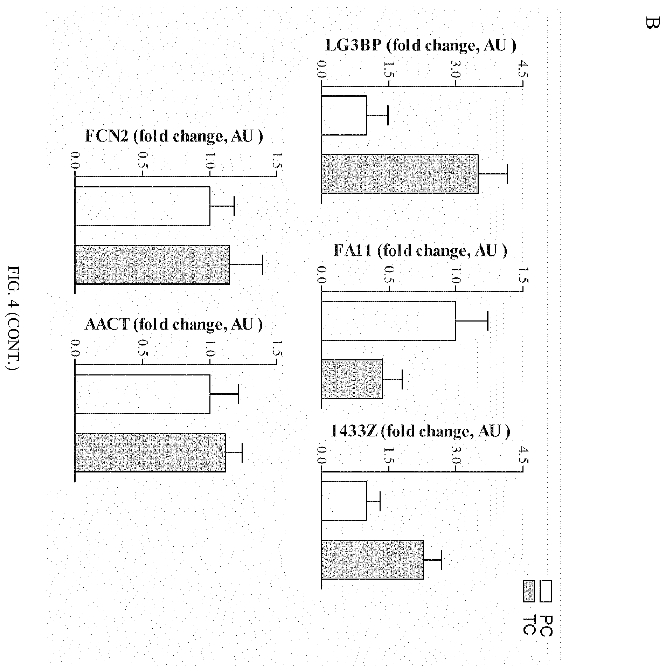

**Figure 4** shows a protein validation by Western Blot analyses. Representative immunoblot of selected proteins in PC or TC using Ponceau S staining as loading control (A). Changes in protein levels between individuals were calculated by immunoblot densitometry; n=4 patients of each group (B). LG3BP, Galectin-3-binding protein; FA11, Coagulation factor XI; 1433Z, 14-3-3 protein zeta/delta; FCN2, Ficolin-2; AACT, Alpha-1-antichymotrypsin; PC, Persistent Controllers; TC, Transient Controllers.

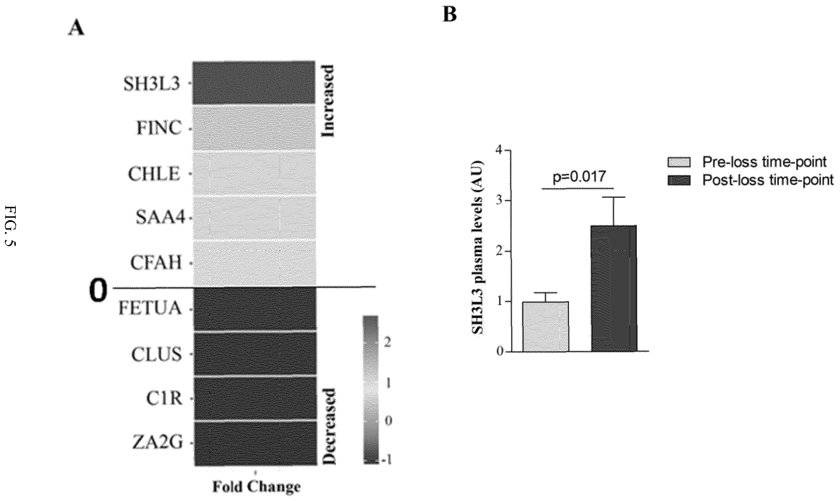

**Figure 5** shows a proteomic analysis comparing TC before and after the loss of natural HIV-1 control. Heatmap representation of the differential and significant protein expression as an effect of the loss of control (A). The scale from light grey (zero fold change) to dark grey (high fold change in absolute values) represents the normalized abundance in arbitrary units. Increased fold changes refer to positive fold changes, whereas decreased fold changes refer to negative fold changes. The most remarkable change was the increased levels of SH3 domain-binding glutamic acid-rich like protein (SH3L3) after the virological progression in TC (B).

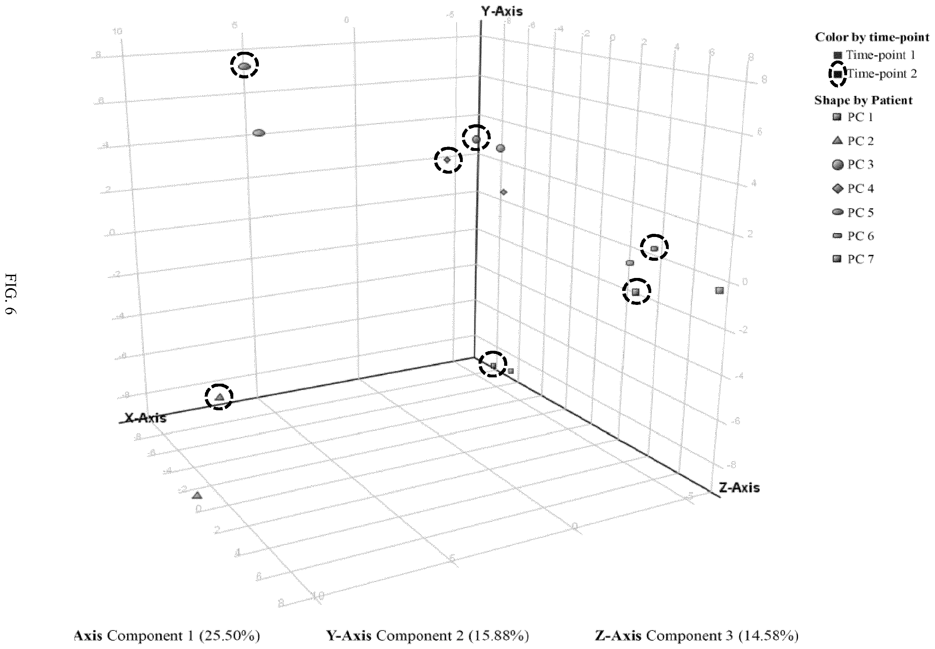

**Figure 6** shows a PCA for paired samples in PC. Each PC is represented with a different shape (n=7), and paired samples from each PC are represented with a square and a square surrounded with a broken line.

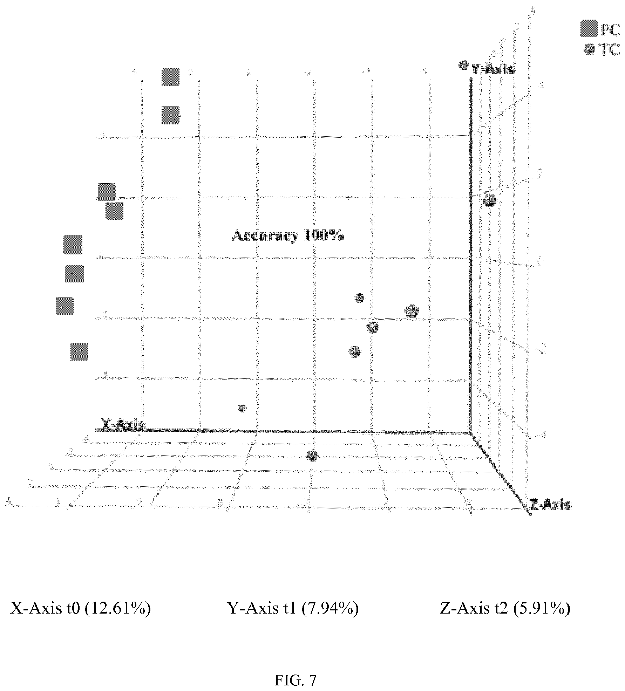

**Figure 7** shows a PLS-DA analysis before the statistical analysis, using all of the quantified proteins between TC (circles, n=8) and PC (squares, n=8).

### DETAILED DESCRIPTION OF THE INVENTION

By comparing the protein expression profile between a set of EC patients along time, the inventors of the present invention have identified a set of markers whose expression is modified in EC subjects that lose control on viral replication 2 years after observing the modification on the expression level of said markers, as compared with EC subjects that did not lose control on viral replication at any time. Thus, the inventors have identified biomarkers the expression of which allows identifying EC patients committed to lose control on viral replication (i.e. future TC subjects) at least 2 years later. Therefore, the proteins identified by the inventors are useful in predicting the prognosis of HIV-infected subjects having an undetectable viral load in the absence of antiretroviral treatment, particularly of EC patients.

### 1- Methods of the invention

### A- Method for determining the prognosis or progression of an HIV-infected subject having an undetectable viral load in the absence of antiretroviral treatment

In a first aspect, the invention relates to an *in vitro* method for determining the prognosis or progression of an HIV-infected subject having an undetectable viral load in the absence of antiretroviral treatment, which comprises:
(i) determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a blood sample from said subject, and
(ii) comparing the expression level of said at least one biomarker with a reference value,
wherein if the expression level of at least one biomarker selected from LG3BP, AACT, FCN2 and 1433Z is increased with respect to the reference value, and/or the level of expression of FA11 is decreased with respect to the reference value, then the subject shows bad prognosis, or
wherein if the expression level of at least one biomarker selected from LG3BP, AACT, FCN2 and 1433Z is decreased or lacks change with respect to the reference value, and/or the level of expression of FA11 is increased or lacks change with respect to the reference value, then the subject shows good prognosis.

The expression "method for determining the prognosis" or "method for determining the progression", or "prognostic method", as used herein, relates to the prediction of a medical outcome, for example, a poor or good outcome (e.g. likelihood of long-term survival, overall survival, disease-specific survival, progression-free survival or disease-free survival). A "negative prognosis", "bad prognosis", or "poor outcome", includes a prediction of relapse, disease progression, mortality or the necessity to change or administer a new medical treatment. In a particular embodiment, a negative prognosis, bad prognosis or poor outcome in the context of the present invention refers to the prediction of a future increase in the HIV viral load in a sample from an HIV-infected subject with undetectable viral load in the absence of antiretroviral treatment in such an amount that the viral load of said patient will appears above the detection limit as defined herein, preferably 50 RNA copies/ml of sample in which the viral load is determined, which is generally a blood sample, preferably a sample of plasma. In another preferred embodiment, it refers to the prediction of a future appearance of symptoms of HIV infection, and associated to HIV-infected patients with a high viral load. A "positive prognosis", or "good prognosis", or "good outcome", refers to the prediction that the disease will stabilize over time or to the prediction that the symptoms associated to the disease will not appear or worsen over time. In a particular embodiment, a positive prognosis, good prognosis, or good outcome in the context of the present invention refers to the prediction that no increase of the HIV viral load in a sample from an HIV-infected subject with undetectable viral load in the absence of antiretroviral treatment will take place in the future, in such an amount that the HIV viral load would reach levels above the detection limit as defined herein. Thus, it refers to the prediction that the HIV viral load level will be maintained below the detection limit as defined herein, i.e., similar to those of an EC patient. In another preferred embodiment, it also includes the prediction that the viral load in the samples from an HIV-infected subject with undetectable viral load in the absence of antiretroviral treatment will increase in the future, but always below the detection limit as defined herein. In another preferred embodiment, it refers to the prediction that the viral load in the samples from an HIV-infected subject with undetectable viral load in the absence of antiretroviral treatment will decrease, or be unchanged, with respect to samples extracted previously, and below the detection limit as defined herein. In another preferred embodiment, it refers to the maintenance of the absence of symptoms of HIV infection, as it is the case of HIV patients with low HIV viral load, such as patients with undetectable viral load, such as EC patients, or HIV patients in the latency phase, or of subjects not infected with HIV.

As it will be understood by those skilled in the art, the prediction, although preferred to be, does not need to be correct for 100% of the subjects to be evaluated. However, the term requires that a statistically significant part of the subjects can be identified as having an increased probability of having a given outcome. The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 50%, at least 60%, at least 70%, at least 80%, at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention. Any parameter which is widely accepted for determining prognosis of a patient can be used in the present invention including, without limitation, overall survival time.

The term *"in vitro",* as used herein, refers to the fact that an experimental protocol or a method is not carried out on the body of a human or animal subject, but on samples isolated from said subject, and already present in a laboratory tool, such as a test tube, dish or plate.

The term "HIV", as used herein, refers to the human immunodeficiency virus (HIV). HIV infection advances through different phases, getting worse over time. HIV attacks the body's immune system, specifically the CD4 cells (T cells). Thus, HIV reduces the number of CD4 cells (T cells) in the infected body, making the patient more likely to get other infections or infection-related cancers. No effective cure currently exists, however, HIV medicines (i.e. antiretrovirals or ARVs) can prevent HIV from advancing to AIDS and also reduce the risk of HIV transmission (the spread of HIV to others). The phases of HIV infection are considered to be the following: (1) acute HIV infection, (2) clinical latency, and (3) AIDS (acquired immunodeficiency syndrome).

HIV can be divided into two major types, HIV type 1 (HIV-1) and HIV type 2 (HIV-2). HIV-1 is related to viruses found in chimpanzees and gorillas living in western Africa, while HIV-2 viruses are related to viruses found in the endangered west African primate sooty mangabey.

### HIV-1

HIV-1 is the most common and pathogenic strain of the virus. There are four distinct HIV-1 groups (M, N, O and P) of which the M group accounts for 90% of infections worldwide. Within the group there are nine phylogenetically distinct subtypes (A-D, F-H, J and K) along with an increasing number of inter-subtype circulating recombinant forms (CRFs). The main HIV-1 subtypes have distinct geographical associations that can provide useful epidemiological information, although there is growing evidence of increasing subtype and inter-subtype HIV-1 genetic diversity in regions previously characterized by specific HIV-1 subtypes. Globally, subtype C is the most prevalent and is strongly associated with sub-Saharan African and Indian populations, followed by subtype A (east Africa) and subtype B (western Europe, United States and Australia); these jointly account for 70% of HIV infections. Other major subtypes (F, H, J and K) have remained stable at low levels, accounting for around 1% of infections worldwide, whilst subtype D has decreased over time. HIV-1 CRFs account for around 17% of infections worldwide; a 50% increase in the number of total global HIV-1 infections between 2000 and 2007. Unique recombinant forms (URFs) account for approximately 4% of all HIV infections globally, though this proportion can increase to as high as 30% of all new infections in regions where multiple subtypes and CRFs co-circulate, known as recombinant hotspots.

HIV-1 group N is a very distinctive form of the virus that has only been identified in a few individuals in Cameroon. N is sometimes referred to as Not-M, Not-O, also sometimes as the "new" group, and is also thought to have originated from a chimpanzee transmission. Subtypes within the HIV-1 N group are not yet clearly defined. Very few isolates have been identified and sequenced.

HIV-1 group O, sometimes referred to as the "outlier" group, like group M contains very diverse viruses, but is still relatively rarely found. It is thought to have originated in a transmission to humans from wild gorilla.

HIV-1 group P was first identified in 2009. The corresponding HIV sequence was reported to have greater similarity to a simian immunodeficiency virus recently discovered in wild gorillas (SIVgor) than to SIVs from chimpanzees (SIVcpz). The virus had been isolated from a Cameroonian woman residing in France who was diagnosed with HIV-1 infection in 2004. Said sequence was in said Group P as "pending the identification of further human cases".

### HIV-2

HIV-2 is closely related to simian immunodeficiency virus endemic in sooty mangabeys (Cercocebus atys atys) (SIVsmm), a monkey species inhabiting the forests of Littoral West Africa. HIV-2 viruses are thought to be less virulent and transmissible than HIV-1 M group viruses, although HIV-2 is known to cause AIDS. HIV-2 has been found to be less pathogenic than HIV-1 and the transmission rate is much lower in HIV-2 than HIV-1. HIV-2 has not been widely recognized outside of Africa.

Both infections can lead to AIDS in affected individuals and both can mutate to develop drug resistance. Disease monitoring in patients with HIV-2 includes clinical evaluation and CD4 cell counts. Many test kits for HIV-1 will also detect HIV-2.

The term "acute HIV infection", as used herein, refers to the initial phase of HIV infection, and it lasts until the body has created antibodies against the virus (i.e. 2-4 weeks after infection with HIV). During this initial stage, the virus is multiplying at a rapid rate, and thus the viral load in the blood of the patient is high. Patients are very contagious at this stage. Typical symptoms of acute infection are flu-like symptoms.

The term "clinical latency", as used herein, refers to the second phase of HIV infection. During this phase, HIV is still active but reproduces at very low levels, and thus patients may not have any symptom, although HIV transmission is still possible. Said phase may last about a decade in the absence of HIV treatment, but upon HIV treatment, said phase may last for several decades. At the end of this phase, viral load starts to increase, and the CD4 cell count starts to decrease. Accordingly, symptoms of the disease arise.

The term "acquired immunodeficiency syndrome", or "AIDS", as used herein, refers to the last and most severe phase of HIV infection. The immune system of patients with AIDS is so damaged that they are affected by an increasing number of severe illnesses, called opportunistic infections.

The expression "HIV-infected subject" refers to a subject, preferably a human being that has been infected by a human immunodeficiency virus (HIV). Therefore it refers to a subject that is going through any of the three stages of the HIV infection indicated in the definition of HIV. HIV is transmitted through: (i) contaminated blood transfusions (usually before 1992), (ii) sharing syringes or needles with someone living with HIV, (iii) contact with blood, semen, vaginal fluids, or anal secretions containing HIV, (iv) pregnancy or breastfeeding if the mother has HIV. Tests currently allowing detecting HIV infection include: (i) determination of the presence of p24 HIV antigen in blood, (ii) determination of the CD4 count and of the HIV RNA viral load, and (iii) determination of the presence of HIV antigen and antibody in blood.

The term "patient" or "subject", as used herein, refers to a human being of any race or age. In the context of the present invention, the patient or subject can be a human being infected with an HIV, a human being infected with HIV but with undetectable viral load in the absence of antiretroviral treatment, or a human being infected with HIV that has been considered an EC at some point.

In a preferred embodiment the HIV-infected subject has been classified as Elite Controller.

The term "HIV Elite controller", "EC", or "EC subject", as used herein, refers to subjects infected with an HIV who are able to maintain viral loads below the detection limit in samples obtained from them in the absence of antiretroviral treatment, preferably in body fluid samples. Said body fluids are those known body fluids that comprise HIV copies in HIV infected subjects (i.e. blood, plasma, semen, vaginal fluids, or anal secretions). In a preferred embodiment, the body fluid is selected from the group consisting of blood, plasma, semen, vaginal fluids or anal secretions, more preferably selected from blood, plasma and semen. Most commonly, said body fluid is blood, preferably plasma. It is generally considered that for an HIV-infected subject to be considered EC, said subject must show a viral load below the detection limit in at least one sample extracted from the subject when the subject had not received antiretroviral treatment in the at least last 6 months, or at least last 12 months or at least last 24 months. In some cases it is also considered that for an HIV to be considered EC the subject must show a viral load below the detection limit in at least 2 samples extracted from the subject at different points of time, in a time interval of at least 1 month, at least 6 months, at least 12 months or at least 24 months, in the absence of antiretroviral treatment. In a preferred embodiment, an EC subject shows a viral load below the detection limit since he is first diagnosed as an HIV infected subject, more preferably in the absence of anti-retroviral treatment. The term "HIV Transient controller", or "TC", as used herein, refers to an EC subject that has lost control on the replication of the HIV. Said subjects are characterized by being classified as EC subjects and spontaneously showing viral loads above the detection limit in at least 2 samples from body fluids of the subject extracted in a time interval of at least 1 month, 6 months or 1 year. Said body fluids are those known body fluids to comprise HIV copies in HIV-infected subjects (i.e. blood, plasma, semen, vaginal fluids, or anal secretions). Most commonly, said body fluid is blood, preferably plasma. In a preferred embodiment, a TC subject spontaneously shows a viral load above the detection limit in the absence of antiretroviral treatment at least 6 months, at least 1 year, at least 1.5 years, at least 2 years, at least 3 years, more preferably at least 1 year, yet more preferably at least 2 years, even yet more preferably at least 3 years after he was first diagnosed as an HIV infected subject with a viral load below the detection limit, more preferably after he was classified as an EC subject. In a more preferred embodiment, a TC subject showed a viral load below the detection limit when first diagnosed as HIV infected subject, more preferably in the absence of antiretroviral treatment. The term "sample", as used herein, refers to biological material isolated from a subject. The sample can derive from or correspond to a representative amount of any fluid or tissue of the subject. Concretely, if the viral load of the subject is to be determined, said sample is from a body fluid known to comprise HIV particles in case the subject has been infected with an HIV, and can be, without limitation, blood, plasma, semen, vaginal fluids, or anal secretions. Generally, the sample is a blood sample, a sample of serum, or more commonly a sample of plasma. If the expression level of a biomarker is to be determined, the sample can derive from a body fluid or a tissue such as, for example, blood, blood plasma, serum, urine, cerebral spinal fluid (CSF), saliva, sputum, deposition, tears, mucus, and sweat. In a particular embodiment, the sample in which the expression level of a biomarker is to be determined is a body fluid sample, preferably a blood sample, more preferably a plasma sample. It is known to comprise cell and/or non-cell material of the subject that can be used to determine the expression level of the biomarkers of the invention, concretely the expression level of at least one biomarker selected from the group consisting of LG3BP, FA11, AACT, FCN2, 1433Z, APOB, CD5L, CXCL7, CNNM4, K1C9, VINC, RET4, CO8G, URP2, ACTB, CBPB2, ICAM1, IBP3 and combinations thereof. The expression level, as it is defined below, refers to the level or quantity of the transcriptional product and/or of the translational product of said biomarkers. Thus, an expert in the field understands that the samples of the invention comprise mRNAs of the genes expressed in the organism of the subject and/or proteins encoded by said genes, and thus they comprise detectable levels of the biomarkers of the invention, in case said biomarkers are expressed in cells of the subject.

The expression "viral load", *"*viral burden", or "viral titer", as used herein, refers to a numerical expression of the quantity of virus, i.e., the number of viral particles, in a volume of a certain sample of a body fluid from a subject. It can be expressed in RNA copies per ml of blood plasma when the sample is blood plasma. A higher viral burden, titer, or viral load often positively correlates with the severity of an active viral infection. Methods to determine the viral load in a sample are known by an expert in the field. Non-limiting examples of techniques useful to determine the viral load in a sample as defined herein from a subject are techniques based on hybridization (e.g., Northern blot analysis or by oligonucleotide microarrays after converting the RNA into a labeled cDNA) and/or amplification (e.g., RT-PCR). Quantitative or semi-quantitative RT-PCR are preferred. Real-time quantitative or semiquantitative RT-PCR is particularly advantageous. Preferably, primer pairs are designed to overlap with sequences specific for HIV RNAs being analyzed, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of HIV RNA, or the number of copies of RNA/ml of sample is measured by quantitative or semi-quantitative RT-PCR or by real-time quantitative or semi-quantitative RT-PCR.

In a preferred embodiment the viral load is measured in a sample from a body fluid from the subject that is tested.

The expression "HIV-infected subject having an undetectable viral load", as used herein, refers to an HIV-infected subject having a viral load that cannot be detected in a body fluid from said subject because it is under the detection limit of the methods used to determine the viral load.

The expression "detection limit", as used herein, refers to the minimal number of viral RNA copies per ml of a sample from a body fluid from a subject that can be detected with the method used to determine the viral load in said sample, wherein the sample is known to comprise viral copies if the body fluid derives from a subject infected with said virus. Regarding HIV infections, said body fluids are, without limitation, blood, plasma, semen, vaginal fluids, or anal secretions. Generally, the body fluid used is blood, more specifically plasma. Viral load levels below the detection limit in a subject are generally associated with higher levels of circulating CD4+ T-lymphocytes than in a subject with viral load levels above the detection limit. The detection limit of the viral load is generally 50 RNA copies/ml of the sample in which the viral load is determined, more commonly in a blood sample, preferably in a sample of plasma.

The expression "the viral load of a patient" or "the viral load of a subject", as used herein, refers to the viral load as defined herein in a sample that derives from a body fluid of said patient or subject, wherein the body fluid is known to comprise viral copies if the subject from whom it derives is infected with said virus. Regarding HIV infections, said body fluids are, without limitation, blood, plasma, semen, vaginal fluids, or anal secretions. Commonly, said sample is generally a blood sample, more specifically a sample of plasma. Thus, in these cases, the viral load of a subject corresponds with the viral load in a sample from the blood or the plasma of said subject.

The expression "a subject with a high viral load", as used herein, refers to a subject wherein the samples in which the viral load is determined show a high viral load, as compared to a reference value of viral load to be specified.

The expression "a subject with low viral load", as used herein, refers to a subject wherein the samples in which the viral load is determined show a low viral load, as compared to a reference value of viral load to be specified.

The expression "reference value of a viral load", as used herein, relates to the viral load in one or more samples used as reference (and referred to as reference samples) used to evaluate the viral load in a sample of interest (referred to as test sample). Said reference and test samples correspond to any of those defined herein when determining the viral load in a subject. As understood by a person skilled in the art, the reference and test samples should derive from the same type of body fluid. Non-limiting examples of a reference sample is a sample obtained from the body fluid of the same subject as the test sample, but extracted in the past, such as at least 2 months, at least 3 months, at least 6 months, at least 9 months, at least 12 months, at least 16 months, at least 24 months before extracting the test sample. The reference sample can also refer to a sample from a subject not infected with HIV, or from a subject with undetectable viral load in the absence of antiretroviral treatment, or from a subject classified as EC and not diagnosed as TC. As a person skilled in the art will understand, the reference viral load can also be the mean viral load determined in a group of reference samples as just defined. Alternatively, the reference value of a viral load can also correspond to the detection limit of viral load.

The first method of the invention is carried out on an HIV-infected subject having an undetectable viral load in the absence of antiretroviral treatment, i.e., on an HIV-infected subject that has not been treated with antiretroviral drugs.

The term "treatment" or "therapy", as used herein, refers to any process, action, application, or the like, wherein a subject or patient, including a human being, with a specific health condition or disorder is provided medical aid with the objective of improving the subject's condition, directly or indirectly, or slowing the progression of a condition or disorder in the subject, or ameliorating at least one symptom of the condition or disorder under treatment. In a preferred embodiment, a treatment or therapy refers to the administration at a specific regimen or dose for a given period of time, of a specific active agent, or a combination of active agents, comprised in a medicament or set of medicaments.

The expression "antiretroviral treatment", as used herein, refers to a treatment based on the administration of antiretroviral drugs characterized in that they suppress viral replication of the HIV, understood as any HIV-1 and/or HIV-2 types. The active agents, referred as antiretroviral agents (ARVs), are generally inhibitors of proteins involved in the replication of the virus, such as inhibitors of the reverse transcriptase and protease. The first HIV specific antiviral medicaments were given as monotherapy in the early 1990's. Since then, the standard of HIV treatment evolved to include the administration of a cocktail or combination of antiretroviral agents. Combined antiretroviral therapy dramatically suppresses viral replication and can reduce the plasma HIV viral load to below the limits of detection of the most sensitive clinical assays (<50 RNA copies/mL) resulting in a significant reconstitution of the immune system as measured by an increase in circulating CD4+ T-lymphocytes.

Non-limiting examples of ARVs are nucleoside reverse transcriptase inhibitors (e.g. Didanosine (ddl), Stavudine (d4T), zidovudine (AZT), TAnofovir (TDF), lamivudine (3TC), Tenofovir alafenamide (TAF), Emtricitabine (FTC) and abacavir (ABC)), non-nucleoside reverse transcriptase inhibitors (e.g. Rilpivirine (RPV), nevirapine (NVP) Etravirine (ETR) and efavirenz (EFV)), integrase inhibitors (Dolutegravir (DTG), Raltegravir (RAL), and Elvitegravir (EVG)) and protease inhibitors (e.g. atazanavir (ATV), Darunavir (DRV), Fosamprenavir (FPV), Indanavir (IDV), Lopinavir (LPV), Ritonavir (RTV), Saquinavir (SQV), Tipranavir (TPV)). Non-limiting examples of antiretroviral treatments comprising a cocktail of ARVs preferably include a combination of two nucleoside reverse transcriptase inhibitors associated with an integrase inhibitor or an unboosted protease inhibibitor such as Dolutegravir, Abacavir and Lamivudine (DTG, ABC, 3TC); Dolutegravir, Emtricitabine, Tenofovir alafenamide (DTG, FTC, TAF), Raltegravir, Emtricitabine, Tenofovir alafenamide (RAL, FTC, TAF), Elvitegravir unboosted with cobicistat, Emtricitabine, Tenofovir alafenamide (EVG/c, FTC, TAF), Darunavir unboosted with cobicistat, Emtricitabine, Tenofovir alafenamide (DRV/c, FTC, TAF), unboosted Darunavir, Emtricitabine, Tenofovir alafenamide (DRV/u, FTC, TAF), Rilpivirina, Emtricitabine, Tenofovir alafenamide (RPV, FTC, TAF).

Each cocktail of ARVs can be administered in a single tablet or in a combination of tablets each of the tablets comprising an ARV or a specific combination of ARVs.

The first step of the method of the invention comprises determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a sample from the subject to be tested.

The term "expression level", as used herein, refers to the expression level of a gene product, more specifically to a measurable quantity of a gene product produced by a specific gene in a specific sample of the subject. The term "gene product", as used herein, refers to a transcriptional product or a translational product, and thus corresponds to the mRNA transcribed from said gene or to the protein encoded by a specific gene. Thus, as understood by the person skilled in the art, the expression level of a gene product corresponds to a quantified amount of the protein encoded by said gene or to the quantified amount of the mRNA transcribed from said gene in a specific sample. When referring to the expression level of a biomarker, it is understood that the gene product is said biomarker, which, as indicated below, can be an mRNA or a protein transcribed from or encoded by the selected genes of the invention.

The level of an mRNA can be determined by methods well known in the art. For example the nucleic acid contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs are designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA, or the mRNA level, is measured by quantitative or semi-quantitative RT-PCR or by real-time quantitative or semi-quantitative RT-PCR. Conventional methods for quantifying mRNA expression levels can be found, for example, in Sambrook et al., 2001 "Molecular cloning: A Laboratory Manual", 3d ed., Cold 35 Spring Harbor Laboratory Press, N.Y., Vol. 1-3.

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labelled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labelled antibodies (primary antibodies) and labelled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins, or determining the protein levels, include, for instance, affinity chromatography techniques or ligand-binding assays. Preferred methods to determine the quantity of a protein, or the protein level in a sample, are liquid chromatography followed by mass-spectrometry, or western blot.

The term "biomarker", as used herein, refers to a gene product from a gene of interest, understood as the transcriptional product or the translational product of a gene of interest (i.e. the mRNA transcribed from said gene or the protein encoded by said gene), the amount of which reflects a specific situation, for example, a condition such as the loss of HIV viral control in an HIV-infected subject that shows undetectable viral load in the absence of antiretroviral treatment, or that is classified as EC. The biomarkers of the invention are primarily (i.e. the primary biomarkers of the invention are) LG3BP, AACT, FCN2, 1433Z, and FA11.

The term "Galectin-3-binding protein", or "LG3BP", as used herein, refers to the protein or the gene encoding the protein with UniProt accession number Q08380 (version 168 of the entry as of 20 June 2018 and/or version 1 of the sequence as of 1 November 1996). The term "Galectin-3-binding protein" includes any of the isoforms that have been described for this protein.

The term "Alpha-1-antichymotrypsin", or "AACT", as used herein, refers to the protein or the gene encoding the protein with UniProt accession number P01011 (version 219 of the entry as of 20 June 2018 and/or version 2 of the sequence as of 1 August 1991). The term "Alpha-1-antichymotrypsin" includes any of the isoforms that have been described for this protein.

The term "Ficolin-2", or "FCN2", as used herein, refers to the protein or the gene encoding the protein with UniProt accession number Q15485 (version 163 of the entry as of 20 June 2018 and version 2 of the sequence as of 15 January 2008). The term "Ficolin-2" includes any of the isoforms that have been described for this protein.

The term "14-3-3 protein zeta/delta", or "1433Z", as used herein, refers to the protein or the gene encoding the protein with UniProt accession number P63104 (version 174 of the entry as of 20 June 2018 and/or version 1 of the sequence as of 13 September 2004). The term "14-3-3 protein zeta/delta" includes any of the isoforms that have been described for this protein.

The term "Coagulation factor XI", or "FA11", as used herein, refers to the protein or the gene encoding the protein with UniProt accession number P03951 (version 215 of the entry as of 20 June 2018 and/or version 1 of the sequence as of 23 October 1986). The term "Coagulation factor XI" includes any of the isoforms that have been described for this protein.

In a preferred embodiment, the method further comprises the determination of the level of expression of a biomarker selected from the group consisting of Apolipoprotein B-100 (APOB), CD5 antigen-like (CD5L), Platelet basic protein (CXCL7), Metal transporter CNNM4 (CNNM4), Keratin, type I cytoskeletal 9 (K1C9), Vinculin (VINC), Retinol-binding protein 4 (RET4), Complement component C8 gamma chain (CO8G), Fermitin family homolog 3 (URP2), Actin, cytoplasmic 1 (ACTB), Carboxypeptidase B2 (CBPB2), Intercellular adhesion molecule 1 (ICAM1), Insulin-like growth factor-binding protein 3 (IBP3) and combinations thereof. Therefore, these biomarkers are secondary biomarkers of the invention.

The term "Apolipoprotein B-100", or "APOB", as used herein, refers to the protein or the gene encoding the protein with UniProt accession number P04114 (version 219 of the entry as of 20 June 2018 and/or version 2 of the sequence as of 13 July 2010). The term "Apolipoprotein B-100" includes any of the isoforms that have been described for this protein.

The term "CD5 antigen-like", or "CD5L", as used herein, refers to the protein or the gene encoding the protein with UniProt accession number O43866 (version 142 of the entry as of 20 June 2018 and/or version 1 of the sequence as of 1 June 1998). The term "CD5 antigen-like" includes any of the isoforms that have been described for this protein.

The term "Platelet basic protein", or "CXCL7", as used herein, refers to the protein or the gene encoding the protein with UniProt accession number P02775 (version 192 of the entry as of 20 June 2018 and/or version 3 of the sequence as of 1 November 1991). The term "Platelet basic protein" includes any of the isoforms that have been described for this protein.

The term "Metal transporter CNNM4", or "CNNM4", as used herein, refers to the protein or the gene encoding the protein with UniProt accession number Q6P4Q7 (version 127 of the entry as of 20 June 2018 and/or version 3 of the sequence as of 3 March 2009). The term "Metal transporter CNNM4" includes any of the isoforms that have been described for this protein.

The term "Keratin, type I cytoskeletal 9", or "K1C9", as used herein, refers to the protein or the gene encoding the protein with UniProt accession number P35527 (version 184 of the entry as of 20 June 2018 and/or version 3 of the sequence as of 16 June 2009). The term term "Keratin, type I cytoskeletal 9" includes any of the isoforms that have been described for this protein.

The term "Vinculin", or "VINC", as used herein, refers to the protein or the gene encoding the protein with UniProt accession number P18206 (version 206 of the entry as of 20 June 2018 and/or version 4 of the sequence as of 23 January 2007). The term "Vinculin" includes any of the isoforms that have been described for this protein.

The term "Retinol-binding protein 4", or "RET4", as used herein, refers to the protein or the gene encoding the protein with UniProt accession number P02753 (version 209 of the entry as of 20 June 2018 and/or version 3 of the sequence as of 29 March 2005). The term "Retinol-binding protein 4" includes any of the isoforms that have been described for this protein.

The term "Complement component C8 gamma chain", or "CO8G", as used herein, refers to the protein or the gene encoding the protein with UniProt accession number P07360 (version 187 of the entry as of 20 June 2018 and/or version 3 of the sequence as of 3 October 2006). The term "Complement component C8 gamma chain" includes any of the isoforms that have been described for this protein.

The term "Fermitin family homolog 3", or "URP2", as used herein, refers to the protein or the gene encoding the protein with UniProt accession number Q86UX7 (version 144 of the entry as of 20 June 2018 and/or version 1 of the sequence as of 1 June 2003). The term "Fermitin family homolog 3" includes any of the isoforms that have been described for this protein.

The term "Actin, cytoplasmic 1", or "ACTB", as used herein, refers to the protein or the gene encoding the protein with UniProt accession number P60709 (version 172 of the entry as of 20 June 2018 and/or version 1 of the sequence as of 1 April 1988). The term "Actin, cytoplasmic 1" includes any of the isoforms that have been described for this protein.

The term "Carboxypeptidase B2", or "CBPB2", as used herein, refers to the protein or the gene encoding the protein with UniProt accession number Q96IY4 (version 147 of the entry as of 20 June 2018 and/or version 2 of the sequence as of 11 January 2011). The term "Carboxypeptidase B2" includes any of the isoforms that have been described for this protein.

The term "Intercellular adhesion molecule 1", or "ICAM1", as used herein, refers to the protein or the gene encoding the protein with UniProt accession number P05362 (version 223 of the entry as of 20 June 2018 and/or version 2 of the sequence as of 21 June 2005). The term "Intercellular adhesion molecule 1" includes any of the isoforms that have been described for this protein.

The term "Insulin-like growth factor-binding protein 3", or "IBP3", as used herein, refers to the protein or the gene encoding the protein with UniProt accession number P17936 (version 205 of the entry as of 20 June 2018 and/or version 2 of the sequence as of 6 March 2007). The term "Insulin-like growth factor-binding protein 3" includes any of the isoforms that have been described for this protein.

The second step of the method of the invention comprises comparing the expression level of said at least one biomarker with a reference value.

The expression "reference value of the expression level of a gene product" or the "reference value of the expression level of a biomarker", as used herein, refers to the expression level of a gene product or of a biomarker in one or more samples used as reference (and referred to as reference samples) used to evaluate the expression level of a biomarker in a sample of interest (referred to as test sample). Said reference and test samples correspond to any of those defined herein when determining the expression level of a gene product or of a biomarker. As understood by a person skilled in the art, the reference and test samples should derive from the same type of body fluid or tissue. Non-limiting examples of a reference sample is a sample obtained from the body fluid of the same subject as the test sample, but extracted in the past, such as at least 2 months, at least 3 months, at least 6 months, at least 9 months, at least 12 months, at least 16 months, at least 24 months before extracting the test sample. The reference sample can also refer to a sample from a subject not infected with HIV, or from a subject with undetectable viral load in the absence of antiretroviral treatment, or from a subject classified as EC and not diagnosed as TC patient or not considered to have probabilities to become a TC patient, or an EC subject that has been considered an EC subject for at least 5 years, at least 8 years, at least 10 years, at least 13 years, at least 15 years, or at least 17 years. As a person skilled in the art will understand, the reference expression level can also consist on the mean of expression levels determined in a group of reference samples as just defined.

According to the method of the invention, if the expression level of at least one biomarker selected from LG3BP, AACT, FCN2 and 1433Z is increased with respect to the reference value, and/or the level of expression of FA11 is decreased with respect to the reference value, then the subject shows bad prognosis, or if the expression level of at least one biomarker selected from LG3BP, AACT, FCN2 and 1433Z is decreased or lacks change with respect to the reference value, and/or the level of expression of FA11 is increased or lacks change with respect to the reference value, then the subject shows good prognosis.

The term "increased expression level", as used herein, refers to the expression level of a gene product of interest, where the level of expression of said gene product as defined herein, is increased at least 5%, at least 10%, at least 20%, at least 30%, at least 40%, at least 50%, at least 60%, at least 70%, at least 80%, at least 90% or at least 100% when compared to a reference value. More preferably, the expression level of a gene product is considered to be increased if it is increased at least 10%, even more preferably if it is increased at least 30% when compared to a reference value.

The term "decreased expression level", as used herein, refers to the expression level of a gene product of interest, where the level of expression of said gene product, as defined herein, is decreased at least 5%, at least 10%, at least 20%, at least 30%, at least 40%, at least 50%, at least 60%, at least 70%, at least 80%, at least 90% or at least 100% when compared to a reference value. More preferably, the expression level of a gene product is considered to be decreased if it is decreased at least 10%, even more preferably if it is decreased at least 30%, when compared to a reference value.

### B. Preferred embodiments of the method of the invention

In a preferred embodiment of the method of the invention, the HIV-infected subject has an undetectable viral load in the absence of administration of antiretroviral treatment to the subject, wherein the viral load is determined in a sample from a body fluid of said subject. More preferably, said subject has not received an antiretroviral treatment in at least the last 6 months, at least the last 9 months, at least the last 12 months, at least the last 16 months, at least the last 24 months, preferably at least the last 6 months, even more preferably the last 12 months, yet more preferably the last 24 months.

In a further preferred embodiment of the method of the invention, the subject with the characteristics indicated above is classified as Elite Controller (EC).

In another embodiment of the method of the invention, a bad prognosis refers to the prediction of an increase in the HIV viral load over time in the subject the prognosis or progression of which is being determined, preferably wherein said increase is detected at least 6 months, at least 1 year, at least 1.5 years, at least 2 years, more preferably at least 1 year, even more preferably at least 2 years after the method of the invention is carried out. Preferably, said increase in the viral load is above the detection limit.

In a preferred embodiment, said increase in the viral load above the detection limit is observed in at least 1 body fluid sample, more preferably in the at least 2 last body fluid samples, extracted from said subject at different time periods, or time points, more preferably in a time interval of at least 1 month, at least 2 months, at least 3 months, at least 6 months, at least 9 months, at least 12 months, at least 16 months, at least 24 months.

In another preferred embodiment, a bad prognosis refers to the prediction that the EC subject of the method of the invention will become a TC subject in the future, preferably at least 6 months, at least 1 year, at least 1.5 years, at least 2 years, more preferably at least 1 year, even more preferably at least 2 years after the method of the invention is carried out.

In another preferred embodiment, a bad prognosis in the method of the invention refers to the prediction of the appearance of physiological symptoms of HIV infection in the future, preferably at least 6 months, at least 1 year, at least 1.5 years, at least 2 years, more preferably at least 1 year, even more preferably at least 2 years after the method of the invention is carried out. Said physiological symptoms are well-known by a skilled person in the art and are, without limitation, lymph nodes inflammation, fever, myalgia, headache, malaise, nonproductive cough, sore throat, rhinitis, fatigue, diarrhea, weight loss, oral candidiasis and appearance of herpes zoster in the subject. Thus, in a preferred embodiment, bad prognosis in said method refers to the appearance of physiological symptoms selected from the list consisting on lymph nodes inflammation, fever, myalgia, headache, malaise, nonproductive cough, sore throat, rhinitis, fatigue, diarrhea, weight loss, oral candidiasis, and appearance of herpes zoster in the subject. Alternatively, a subject with an HIV infection or a TC subject may present no physiological symptoms of HIV infection or associated to a TC subject, although the subject is infected with HIV and shows a viral load in a sample of the subject above the detection limit, or is a TC subject. In this case, the subject the prognosis of which is being determined may present no physiological symptoms of HIV infection or associated to a TC subject, although the prognosis obtained with the method of the invention is a bad prognosis.

In another preferred embodiment of the method of the invention a good prognosis refers to the prediction that the subject, the prognosis or progression of which is being determined, will remain with undetectable viral load levels in the absence of antiretroviral treatment, and preferably remain or become classified as EC, in the future. In a further preferred embodiment, said prognosis applies to the 6 months, the 12 months, the 1 year, the 1.5 years, the 2 years, more preferably the 1 year, even more preferably the 2 years after the method is carried out.

In another embodiment of the method of the invention, a good prognosis refers to the maintenance of the absence of symptoms of HIV infection such as those indicated above, and as it is the case of HIV patients with HIV viral load below the detection limit, such as of EC patients, or of subjects not infected with HIV.

In a preferred embodiment the detection limit of the viral load is of less than 500 HIV RNA copies/ml, less than 300 HIV RNA copies/ml, less than 200 HIV RNA copies/ml, less than 100 HIV RNA copies/ml, less than 75 HIV RNA copies/ml, less than 50 HIV RNA copies/ml of the body fluid sample in which the viral load is determined. More preferably, the detection limit of the viral load is 50 HIV RNA copies/ml of the body fluid sample in which the viral load is determined. Furthermore, a reduction of the viral load in a subject below the detection limit is associated to an increase in circulating CD4+ T-lymphocytes in the subject.

In another preferred embodiment, the sample in which the viral load is determined in any of the embodiments of the method of the invention is from a body fluid, i.e., it is a body fluid sample, wherein the body fluid is preferably selected from the group consisting of blood, plasma, semen, vaginal fluids, and anal secretions. Preferably, the body fluid is blood, even more preferably plasma. Accordingly, as understood by an expert in the field, the detection limit is in a preferred embodiment of 50 HIV RNA copies/ml of blood, or in a more preferred embodiment of 50 HIV RNA copies/ml plasma.

In a preferred embodiment, the subject of the method of the invention is addressed is a human being. Thus, the subject to which the methods of the invention are addressed is preferably a human subject infected with the HIV and with undetectable viral load in the absence of antiretroviral treatment, preferably classified as EC.

In a particular embodiment, the subject of the method of the invention is infected with any of the HIV types indicated in section 1-A. In an embodiment, the subject is infected with HIV-2. In a more preferred embodiment, the subject to which the methods of the invention are addressed is infected with HIV-1, preferably with any of the HIV-1 groups indicated in section 1-A, even more preferably with the HIV-1 group M.

The method of the invention comprises determining the expression level of at least one biomarker selected from the group consisting of LG3BP, FA11, AACT, FCN2, 1433Z and a combination thereof in a sample from the subject of the invention. More preferably, it comprises determining the expression level of at least 2, at least 3, at least 4, or the 5 biomarkers selected from said group. In more preferred embodiment, the method of the invention comprises determining the level of expression of LG3BP, AACT, FCN2, 1433Z and FA11.

In another preferred embodiment, the method of the invention comprises determining the expression level of LG3BP in said sample. In another embodiment, it comprises determining the expression level of LG3BP and at least one, at least 2, or 3 biomarkers selected from the group consisting of FA11, AACT, FCN2 and 1433Z in said sample.

In another preferred embodiment it comprises determining the expression level of LG3BP and at least one biomarker selected from the group consisting of 1433Z and FA11. More preferably, it comprises determining the expression level of LG3BP and 1433Z, even more preferably the expression level of LG3BP and FA11. In another preferred embodiment it comprises determining the expression level of LG3BP and 1433Z in combination with at least one or at least 2 additional biomarkers selected from the group consisting of FA11, AACT and FCN2 in said sample.

In another preferred embodiment, it comprises determining the expression level of the gene 1433Z in said sample. In another preferred embodiment it comprises determining the expression level of the gene 1433Z in combination with at least one or at least 2 biomarkers selected from the group consisting of FA11, AACT and FCN2 in said sample.

In another preferred embodiment, it comprises determining the expression level of the gene FA11 in said sample. In another preferred embodiment it comprises determining the expression level of the gene FA11 in combination with at least one or at least 2 biomarker/s selected from the group consisting of 1433Z, AACT and FCN2 in said sample.

In another preferred embodiment it comprises determining the expression level of LG3BP and FCN2 in said sample. In another preferred embodiment it comprises determining the expression level of LG3BP and FCN2 in combination with at least one or at least 2 additional biomarkers selected from the group consisting of FA11, AACT and 1433Z in said sample.

In another preferred embodiment, it comprises determining the expression level of the gene FCN2 in said sample. In another preferred embodiment it comprises determining the expression level of the gene FCN2 in combination with at least one or at least 2 biomarkers selected from the group consisting of FA11, AACT and 1433Z in said sample.

In another preferred embodiment, the method of the invention comprises determining the expression level of at least one biomarker selected from the group consisting of APOB, CD5L, CXCL7, CNNM4, K1C9, VINC, RET4, CO8G, URP2, ACTB, CBPB2, ICAM1, IBP3, and a combination thereof in the same sample of the subject of the invention. In a further preferred embodiment, the expression level of at least one biomarker selected from said group is determined in addition to the expression level of those biomarkers indicated in any of the embodiments of the method of the invention. In another preferred embodiment, the method of the invention comprises determining the expression level of APOB, CD5L, CXCL7, CNNM4, K1C9, VINC, RET4, CO8G, URP2, ACTB, CBPB2, ICAM1, IBP3. In a further preferred embodiment, the expression level of said group of biomarkers is determined in addition to the expression level of those biomarkers indicated in any of the embodiments of the method of the invention.

The abbreviations for all the proteins referred to in the methods of the invention are listed in Table 1.

**Table 1. List of the differentially plasma protein levels comparing PC and TC at pre-loss of control time-point.**

| **Swiss-Prot Accessio n Number** | **Swiss-Prot Entry** | **Protein description** | **Gene name** | ***p*** |
|---|---|---|---|---|
| Q08380 | LG3BP_HUMAN | Galectin-3-binding protein | LGALS3BP | 2.83E-04 |
| P03951 | FA11_HUMAN | Coagulation factor XI | F11 | 2.59E-03 |
| Q15485 | FCN2_HUMAN | Ficolin-2 | FCN2 | 4.53E-03 |
| P01011 | AACT_HUMAN | Alpha-1-antichymotrypsin | SERPINA3 | 1.06E-02 |
| P63104 | 1433Z_HUMAN | 14-3-3 protein zeta/delta | YWHAZ | 1.21E-02 |
| Q96IY4 | CBPB2_HUMAN | Carboxypeptidase B2 | CPB2 | 1.21E-02 |
| Q86UX7 | URP2_HUMAN | Fermitin family homolog 3 | FERMT3 | 1.93E-02 |
| O43866 | CD5L_HUMAN | CD5 antigen-like | CD5L | 2.47E-02 |
| P07360 | CO8G_HUMAN | Complement component C8 gamma chain | C8G | 3.05E-02 |
| P02753 | RET4_HUMAN | Retinol-binding protein 4 | RBP4 | 3.32E-02 |
| P35527 | K1C9_HUMAN | Keratin, type I cytoskeletal 9 | KRT9 | 3.33E-02 |
| P18206 | VINC_HUMAN | Vinculin | VCL | 3.62E-02 |
| Q6P4Q7 | CNNM4_HUMAN | Metal transporter CNNM4 | CNNM4 | 3.69E-02 |
| P60709 | ACTB_HUMAN | Actin, cytoplasmic | ACTB | 3.92E-02 |
| P05362 | ICAM1_HUMAN | Intercellular adhesion molecule 1 | ICAM1 | 3.99E-02 |
| P04114 | APOB_HUMAN | Apolipoprotein B-100 | APOB | 4.05E-02 |
| P17936 | IBP3_HUMAN | Insulin-like growth factor-binding protein 3 | IGFBP3 | 4.23E-02 |
| P02775 | CXCL7_HUMAN | Platelet basic protein | PPBP | 4.73E-02 |

Differences between groups were calculated using a Student's t test.

In a preferred embodiment, the sample wherein the expression level of the biomarker is determined is from a body fluid or a tissue of the subject the prognosis or progression of which is being determined. Preferably said sample is from blood, even more preferably from plasma.

According to the method of the invention, an increased expression level of any of the biomarkers LG3BP, AACT, FCN2 or 1433Z, and/or a decrease of the expression level of the biomarker FA11, with respect to a reference value is indicative of a bad prognosis, whereas no change or a decreased expression level of any of the biomarkers LG3BP, AACT, FCN2 or 1433Z, and/or no change or an increase of the expression level of the biomarker FA11, with respect to a reference value is indicative of a good prognosis. Thus, in a preferred embodiment, when determining the expression level of one biomarker or of a subset of biomarkers referred in any of the embodiments indicated above in this section, if said biomarker or subset of biomarkers is comprised in the group of biomarkers LG3BP, AACT, FCN2 or 1433Z, said expression level is indicative of a bad prognosis when it is increased with respect to their corresponding reference value, and it is indicative of a good prognosis when it does not change or is decreased with respect to their corresponding reference value. In another preferred embodiment, when determining the expression level of FA11, its expression level is indicative of bad prognosis in case it is decreased with respect to a reference value and it is indicative of good prognosis in case it is increased or unchanged with respect to said reference value.

In another embodiment, when determining the expression level of any of the biomarkers APOB, CD5L, CXCL7, CNNM4, K1C9, VINC, RET4, CO8G, URP2, ACTB, CBPB2, ICAM1 and/or IBP3, if the expression level of any of the biomarkers APOB, CD5L, CXCL7, CNNM4, VINC, URP2, or ACTB is increased with respect to their corresponding reference value and/or the expression level of any of the biomarkers K1C9, RET4, CO8G, CBPB2, ICAM1, or IBP3 is decreased with respect to their corresponding reference value, said expression levels are indicative of a bad prognosis.

In another preferred embodiment, when determining the expression level of any of the biomarkers APOB, CD5L, CXCL7, CNNM4, K1C9, VINC, RET4, CO8G, URP2, ACTB, CBPB2, ICAM1 and/or IBP3, if the expression level of any of the biomarkers APOB, CD5L, CXCL7, CNNM4, VINC, URP2, or ACTB does not change or is decreased with respect to their corresponding reference value and/or the expression level of any of the biomarkers K1C9, RET4, CO8G, CBPB2, ICAM1, IBP3 does not change or is increased, with respect to their corresponding reference value, said expression levels are indicative of a good prognosis.

In a preferred embodiment, good prognosis is determined in the method of the invention when the expression level of all the biomarkers whose expression level is determined is that associated to good prognosis in any of the embodiments of the method of the invention. In another embodiment, bad prognosis is determined in the method of the invention when the expression level of all the biomarkers whose expression level is determined is that associated to bad prognosis in any of the embodiments of the method of the invention.

In a preferred embodiment, the reference value of the expression level of the biomarkers of the method of the invention is the expression level of said biomarkers in a reference sample. In a preferred embodiment said reference sample is a sample from one or more HIV-infected subjects that have not shown a viral load above the detection limit in at least the last 5 years, at least the last 8 years, at least the last 10 years, at least the last 12 years, at least the last 13 years, at least the last 14 years, at least the last 15 years, at least the last 20 years in the absence of antiretroviral treatment, more preferably in at least the last 5 years, even more preferably in at least the last 10 years, yet more preferably in at least the last 13 years in the absence of antiretroviral treatment. Said reference sample is preferably that of an EC subject classified as EC for at least 5 years, at least 8 years, at least 10 years, at least 12 years, at least 13 years, at least 14 years, at least 15 years, at least 20 years, more preferably for at least 5 years, even more preferably for at least 10 years, yet more preferably for at least 13 years. The reference sample can also correspond to a sample extracted from the subject the prognosis or progression of which is being determined, but extracted in the past, at least 2 months, at least 3 months, at least 6 months, at least 9 months, at least 12 months, at least 16 months, at least 24 months, more preferably at least 24 months before extracting the test sample and carrying the method of the invention, if the subject was already infected with HIV and its viral load was already below the detection limit at that time. In another embodiment, the reference sample refers to a sample from a subject not infected with HIV.

In another preferred embodiment, the reference value of the expression level of a biomarker is the mean of the expression levels of said biomarker in a group of samples wherein each sample is any of the reference samples indicated above.

In a preferred embodiment, the biomarkers of any of the embodiments of the method of the invention are the mRNAs transcribed from the corresponding genes. Thus, in a preferred embodiment, the expression level of the biomarkers refers to the expression level of the mRNAs transcribed from the corresponding genes. Techniques to determine the mRNA expression levels in the corresponding samples are well known by a person skilled in the art. Non-limiting examples of said techniques are indicated in the definition of "expression level" in section 1-A. In a preferred embodiment, the determination of the expression level of the biomarker is carried out by determining the levels of the mRNA of the corresponding gene, preferably by quantitative or semi-quantitative RT-PCR (reverse transcription PCR) or by real-time quantitative or semi-quantitative RT-PCR (reverse transcription PCR), more preferably by quantitative or semi-quantitative RT-PCR, even more preferably by quantitative RT-PCR. Conventional methods for quantifying mRNA expression levels can be found, for example, in Sambrook et al., 2001 "Molecular cloning: A Laboratory Manual", 3d ed., Cold 35 Spring Harbor Laboratory Press, N.Y., Vol. 1-3.

In a more preferred embodiment, the biomarkers of any of the embodiments of the method of the invention are the proteins encoded by the corresponding genes. Thus, in a preferred embodiment, the expression level of the biomarkers refers to the expression levels of the proteins encoded by the corresponding genes. Techniques to determine the protein expression levels in the corresponding samples are well known by a person skilled in the art. Non-limiting examples of said techniques are indicated in the definition of "expression level" in section 1-A. In a preferred embodiment, the determination of the expression level of the biomarker is carried out by determining the levels of the protein encoded by the corresponding gene, preferably by liquid chromatography followed by mass-spectrometry, even more preferably by western blot.

### 2- Kits, and uses of the kits or reagents of the invention

In a second aspect, the invention relates to a kit comprising reagents adequate for the determination of the expression level of at least one biomarker selected from the group consisting of LG3BP, AACT, FCN2, 1433Z and FA11.

In a third aspect, the invention relates to the use of a kit according to the second aspect or of a reagent capable of determining the expression level of at least one biomarker selected from the group consisting of LG3BP, AACT, FCN2, 1433Z and FA11 for determining the prognosis or progression of an HIV-infected subject having an undetectable viral load in the absence of antiretroviral treatment.

The expression "kit" or "kit-of parts", as used herein, refers to an entity of physically separated components, which are intended for individual use, but in functional relation to each other. The kit of the invention is a product that contains the different reagents needed to carry on the different methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagents adequate for the determination of the expression level" or "reagent capable of determining the expression level", as used herein, refers to reagents capable of determining the transcriptional product of the gene (mRNA in case of protein-coding genes) or the protein encoded by said genes.

The expression "constitutive gene", as used herein, refers to a gene that is transcribed continually at a relatively constant rate as opposed to a facultative gene, which is only transcribed when needed. As well-known by a person skilled in the art, the expression level of a "constitutive gene" can be used as a reference point in experiments to measure the expression rates of other genes. Commonly used constitutive genes are "housekeeping genes", which are required to maintain basic cellular function and so they are typically expressed in all cell types of an organism at a constant rate. Examples of "constitutive genes" are well-known by a person skilled in the art. Non-limiting examples of constitutive genes are actin, GAPDH and ubiquitin.

All the terms described elsewhere herein are equally applicable to the second and third aspects of the invention.

### Preferred embodiments of the kits and uses of the kits or reagents of the invention

In a preferred embodiment, the reagents of the kit of the invention are adequate for the determination of the expression level of at least two biomarkers selected from the group consisting of LG3BP, AACT, FCN2, 1433Z and FA11, preferably for the determination of the expression level of at least LG3BP, 1433Z and FA11, more preferably at least LG3BP and 1433Z, even more preferably at least LG3BP and FA11, yet more preferably at least LG3BP and FCN2.

In a more preferred embodiment, the reagents of the kit of the invention are adequate for the determination of the expression level of any of the biomarkers whose expression level is determined in any of the embodiments of the method of the invention.

In a particular embodiment, the kit of the invention further comprises reagents adequate for the determination of the expression level of one or more reference biomarkers and/or constitutive genes. In a preferred embodiment, the constitutive genes are selected from the group consisting of actin, GAPDH and ubiquitin. The expression level of any of the constitutive genes consists on the expression level of the mRNAs of the corresponding constitutive genes, or on the expression level of the proteins encoded by the corresponding constitutive genes.

In another preferred embodiment, the reagents of the kit of the invention are reagents adequate for the determination of the expression levels of mRNAs. Therefore, said reagents are those required for developing the methods adequate to determine the mRNA levels in a sample of the invention and indicated in the definition of "expression level" in section 1-A. In a more preferred embodiment, said reagents are those required for determining the mRNA levels of the corresponding genes by a quantitative or semi-quantitative RT-PCR (reverse transcription PCR) or by a real-time quantitative or semi-quantitative RT-PCR (reverse transcription PCR), more preferably by a quantitative or semi-quantitative RT-PCR, even more preferably by quantitative RT-PCR. In an even more preferred embodiment, the reagents are primer pairs which are capable of specifically amplifying the mRNAs whose expression is to be determined with the kit of the invention, and/or probes which specifically hybridize to said mRNAs, i.e., mRNAs transcribed from genes selected from the group consisting of LG3BP, AACT, FCN2, 1433Z, FA11, one or more constitutive genes and combinations thereof.

In a more preferred embodiment the reagents of the kits of the invention are reagents adequate for the determination of the expression levels of proteins. Therefore, said reagents are those required for developing the methods adequate to determine the protein levels in a sample of the invention and indicated in the definition of "expression level" in section 1-A. In a more preferred embodiment, said reagents are antibodies with the capacity for binding specifically to the proteins whose expression is to be determined with the kit of the invention (or to fragments thereof containing the antigenic determinants). In a more preferred embodiment the kit also contains those reagents required for subsequent quantification of the resulting antigen-antibody complexes. The antibodies can be monoclonal, polyclonal or fragments thereof, Fv, Fab, Fab' and F(ab')2, scFv, diabodies, triabodies, tetrabodies and humanized antibodies. Similarly, the antibodies may be labelled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. In another preferred embodiment, the reagents of the kit of the invention are non-labelled antibodies (primary antibodies) specifically binding to the proteins the expression of which is being determined with the kit of the invention. In another embodiment the reagents of the kit are labelled antibodies (secondary antibodies).

In a preferred embodiment, the reagents adequate for the determination of the expression levels of said biomarkers comprise at least 10% of the total amount of reagents forming the kit. In further embodiments, the reagents adequate for the determination of the expression levels of one or more biomarkers comprise at least 15%, at least 20%, at least 30%, at least 40%, at least 50%, at least 55% at least 60%, at least 65%, at least 70%, at least 75%, at least 80%, at least 90%, at least 95%, at least 96%, at least 97%, at least 98% or at least 99% of the total amount of reagents forming the kit. In a further preferred embodiment, said percentage also includes the reagents required to determine the expression level of the constitutive genes. Accordingly, a skilled person in the art will understand that in case the kit also comprises reagents to determine the expression level of constitutive genes, the reagents of the kit adequate for the determination of the expression level of the biomarkers and of the constitutive genes comprise at least 10% of the total amount of reagents forming the kit, and in a further embodiment, they comprise at least any of the percentages indicated above.

In a preferred embodiment, the kit of the invention, and the reagents described in any of the embodiments in the context of the kit of the invention, are used to develop any of the embodiments of the first aspect of the invention.

All the embodiments described elsewhere herein are equally applicable to the second and third aspects of the invention.

The invention also relates to:
[1]. An *in vitro* method for determining the prognosis or progression of an HIV-infected subject having an undetectable viral load in the absence of antiretroviral treatment, which comprises:
   (i) determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a sample from said subject, and
   (ii) comparing the expression level of said at least one biomarker with a reference value,
   wherein if the expression level of at least one biomarker selected from LG3BP, AACT, FCN2 and 1433Z is increased with respect to the reference value, and/or the level of expression of FA11 is decreased with respect to the reference value, then the subject shows bad prognosis, or
   wherein if the expression level of at least one biomarker selected from LG3BP, AACT, FCN2 and 1433Z is decreased or lacks change with respect to the reference value, and/or the level of expression of FA11 is increased or lacks change with respect to the reference value, then the subject shows good prognosis.
[2]. The method according to [1], wherein the viral load is measured in a sample from a body fluid from said subject in the absence of an antiretroviral treatment for at least the last 6 months.
[3]. The method according to any one of [1] or [2], wherein the subject is classified as Elite Controller (EC).
[4]. The method according to any one of [1] to [3], wherein bad prognosis is characterized by an increase of the viral load over time above a detection limit in at least the last two body fluid samples extracted from the subject at different periods of time.
[5]. The method according to [4], wherein the last two body fluid samples extracted from the subject are extracted in an interval of at least 1 year.
[6]. The method according to any one of [1] to [5], wherein the detection limit of the viral load is 50 HIV RNA copies/ml of the body fluid sample in which the viral load is determined.
[7]. The method according to any one of [1] to [6] wherein the body fluid sample in which the viral load is determined is a blood sample.
[8]. The method according to any one of [1] to [7], wherein the reference value is the expression level of the corresponding biomarker determined in a sample from one or more HIV-infected subjects that have not shown a viral load above the detection limit in at least the last 5 years in the absence of antiretroviral treatment.
[9]. The method according to any one of [1] to [8] wherein the sample in which the expression level of the at least one biomarker is determined is a blood sample.
[10]. The method according to [9], wherein the blood sample is a plasma sample.
[11]. The method according to any one of [1] to [10], wherein the determination of the expression level of the biomarker is carried out by determining the levels of the mRNA of the corresponding gene or by determining the levels of the protein encoded by said gene.
[12]. The method according to any one of [1] to [11], wherein the expression level of said biomarker is determined by Western blot or liquid chromatography associated to mass spectrometry.
[13]. The method according to any one of [1] to [12], wherein the HIV-infected subject is a subject infected by HIV-1.
[14]. The method according to any one of [1] to [13], comprising determining the level of expression of LG3BP, AACT, FCN2, 1433Z and FA11.
[15]. The method according to any one of [1] to [14], further comprising the determination of the level of expression of a biomarker selected from the group consisting of APOB, CD5L, CXCL7, CNNM4, K1C9, VINC, RET4, CO8G, URP2, ACTB, CBPB2, ICAM1, IBP3 and combinations thereof.
[16]. The method according to any one of [1] to [15], wherein the subject is a human.
[17]. A kit comprising reagents adequate for the determination of the expression level of at least one biomarker selected from the group consisting of LG3BP, AACT, FCN2, 1433Z and FA11.
[18]. The kit according to [17], wherein said kit or assay device comprises reagents adequate for the determination of the expression level of at least two biomarkers selected from the group consisting of LG3BP, AACT, FCN2, 1433Z and FA11.
[19]. The kit according to any one of [17] or [18], wherein the biomarkers are mRNAs and the reagents adequate for the determination of the expression level are primer pairs which are capable of specifically amplifying said biomarkers or probes which specifically hybridize to said biomarker.
[20]. The kit according to any one of [17] or [18], wherein the biomarkers are proteins and the reagents adequate for the determination of the expression level are antibodies capable of specifically binding to said biomarker.
[21]. The kit according to any one of [17] to [20], wherein said reagents comprise at least 10% of the reagents present in the kit.
[22]. The kit according to any one of [17] to [21], further comprising reagents adequate for the determination of the expression level of one or more reference biomarkers and/or reagents adequate for the determination of the expression level of one or more constitutive genes.
[23]. Use of a kit according to any one of [17] to [22] or of a reagent capable of determining the expression level of at least one biomarker selected from the group consisting of LG3BP, AACT, FCN2, 1433Z and FA11 for determining the prognosis or progression of an HIV-infected subject having an undetectable viral load in the absence of antiretroviral treatment.

The invention is defined below by virtue of the following examples which are to be construed as merely illustrative and not limitative of the scope of the invention.

### EXAMPLES

### EXAMPLE 1. Materials and methods.

### Patients and study design

EC were defined as subjects with VL determinations below the detection limit (<50 HIV-1-RNA copies/mL) in the absence of antiretroviral treatment for at least 12 months (Dominguez-Molina B, et al., Clin. Infect. Dis. 2016; 62:1304-1309). Subjects were included based on frozen plasma sample availability according to the study design (Figure 1). Samples were received, processed and stored at the Spanish HIV HGM BioBank belonging to the AIDS Research Network (RIS) (Garcia-Merino I et al., Retrovirology 2009; 6:1-5) and data were registered in the RIS cohort of the HIV Controllers Study Group (ECRIS). The cohort's characteristics were previously described in detail (Dominguez-Molina B, et al., Clin. Infect. Dis. 2016; 62:1304-1309). A total of 16 subjects were retrospectively selected and analyzed: eight were EC who had experienced loss of spontaneous virological HIV-1 control (at least two consecutive measurements of VL above the detection limit over 12 months), named Transient Controllers (TC), and another group of eight EC who persistently maintained virological control during the same follow-up period, named Persistent Controllers (PC) (see study design in Figure 1). In TC, up to four determinations were assessed: at one and two years before the loss of virological control, called the "pre-loss-of-control period", (-T1 and -T2, respectively), and up to two more in the "post-loss-of-control period" including the closest time point (T0) and one year (+T1) after the loss of virological control. To be included in the study, at least the -T2, -T1 and T0 samples were required in the TC group. In PC, up to two determinations were performed at one year intervals. All subjects provided their informed consent, and the protocols were approved by the institutional ethical committees.

### Proteomic analysis

For proteomic analysis, the seven most abundant plasma proteins (albumin, IgG, antitrypsin, IgA, transferrin, haptoglobin and fibrinogen) were depleted using a Human7 MARS cartridge (Agilent) following the manufacturer's protocol and the flow through fractions were concentrated and buffer exchanged to approximately 100 µl of 50 mM ammonium bicarbonate (ABC) using 5K MWCO spin columns (Agilent). Then, 65 µg of protein (quantified by Bradford's method) were incubated at 96°C for 3 minutes, reduced with 4 mM 1.4-Dithiothreitol (DTT) for 25 minutes at 56°C and alkylated with 8 mM iodoacetamide (IAA) for 30 min at 25°C in the dark. Afterwards, samples were digested overnight (pH 8.0, 37°C) with sequencing-grade trypsin (Promega) at an enzyme:protein ratio of 1:50. Peptides were desalted on a C18 SPE column (BondElut, Agilent) and labelled with TMT 10-plex reagents (Thermo Fisher) following the manufacturer's instructions. To normalize all samples in the study, a pool containing all of the samples was labeled with TMT-126 tag and included in each TMT batch.

Multiplexed samples were on-line fractionated in a strong cation exchange nano-column (350 µm x 3.5 cm; 3.5 µm, Agilent) coupled to a C18 trap nano-column (100 µm x 2 cm; 5 µm, Thermo Fisher, USA) and a C-18 analytical nano-column (75 µm x 15 cm; 3 µm, Nikkyo Technos, Japan) on an EASY-II nanoLC chromatograph (Thermo Fisher) by a gradient salt pulsed sequential elution using ammonium acetate (0, 12.5, 25, 37.5, 50, 75, 100, 250 and 500 mM NH4AcO) followed by a continuous water-acetonitrile (0.1% formic acid) elution gradient at 300 nl/min.

Mass spectrometry analyses were performed on an LTQ-Orbitrap Velos Pro (Thermo Fisher) by acquiring an enhanced FT-resolution spectrum (R=30,000 FHMW) followed by two data-dependent MS/MS scan events from the most intense ten-parent ions with a charge state rejection of one and a dynamic exclusion of 0.5 min. Thus, an HCD fragmentation (40% NCE) with FT-MS/MS acquisition (R=15,000 FHMW) was conducted for peptide quantification, followed by a CID fragmentation (35% NCE) from the same parent ions with IT-MS/MS acquisition for peptide identification.

Protein identification/quantification was performed on Proteome Discoverer software v.1.4.0.288 (Thermo Fisher) by Multidimensional Protein Identification Technology (MudPIT). For protein identification, the MS and MS/MS spectra were analyzed using Mascot search engine (v.2.5) with SwissProt_2016_07.fasta database (551,705 entries), restricted for human taxonomy (20,198 sequences) and assuming trypsin digestion. Two missed cleavages and an error of 0.02 Da for FT-MS/MS, 0.8 Da for IT-MS/MS and 10.0 ppm for a FT-MS spectra were allowed. The TMT-10plex was set as quantification modification, oxidation of methionine and acetylation of N-termini were set as dynamic modifications, whereas carbamidomethylation of cysteine was set as static modification. The false discovery rate (FDR) and protein probabilities were calculated by Percolator. For protein quantification, the ratios between each TMT label against the 126-TMT label were used, and the quantification results were normalized based on the protein median and Log2 transformed for statistical analysis.

### Western blot

Protein samples were submitted to SDS-PAGE, transferred to nitrocellulose membranes. The membrane was then blocked, incubated with a primary antibody followed by an appropriate secondary antibody conjugated with horseradish peroxidase (HRP) and developed by chemiluminescence using Versadoc (Bio-Rad Laboratories). Finally, the proteins were quantified by ImageJ software. The primary antibodies used were: LGALS3BP (ab67353), Ficolin 2 (ab56225), Anti-alpha 1 Antichymotrypsin (EPR14118(B)), Factor XI (MM0193-7C38) all from Abcam and 14-3-3 z/d (#7413, Cell Signaling Technology). Secondary antibodies were polyclonal goat anti-rabbit (Pierce) and polyclonal goat anti-mouse (Pierce).

### Protein function and pathway analysis

Protein function was elucidated by a SWISS-PROT database (http://www.expasy.org) search. Interaction between the differentially expressed proteins and HIV proteins was investigated with the HIV interaction database (http://www.ncbi.nlm.nih.gov/RefSeq/HIVInteractions). Signaling pathway analysis was defined with STRING v.10 (http://string.embl.de/) using proteins identified in this work and data from the SWISS-PROT function annotation as input.

### Statistical analysis

To find the significant protein changes between the different conditions Mass Profiler Professional software v.14.5 (Agilent Technologies) was used. For statistical purposes, only those proteins that were quantified in more than 70% of the samples and in at least one of the groups were considered. Firstly, a paired Student's t-test for PC samples was performed to check if a time effect existed in this group. Next, a Student's t-test between PC and TC in the pre-loss-of-control-period was performed to find early biomarkers of control loss. Finally, a paired t-test between pre- and post-loss of the control period in the PC group was conducted to find protein changes over time. In all of the comparisons, a multivariate analysis, such as Hierarchical Clustering Analysis (HCA) and Principal Component Analysis (PCA), was performed. To select and evaluate the performance of the potential biomarker, Random Forest analysis, PCA and characteristic operating curves (ROC) were conducted using the 'R' program (http://cran.r-project.org) and the SPSS 21.0 package (IBM, Madrid, Spain). P-values <0.05 were considered statistically significant.

### Laboratory determinations

Laboratory evaluations were performed at the Laboratory of Joan XXIII University Hospital in Tarragona, IISPV, Rovira i Virgili University (Spain) and at the Center for Omics Sciences.

CD4+ and CD8+ T-cell absolute counts (cells/mm³) were determined in fresh whole blood using an Epic XL-MCL flow cytometer (Beckman-Coulter, Brea, CA) and plasma HIV-1 RNA concentration was measured by quantitative polymerase chain reaction (COBAS Ampliprep/COBAS Taqman HIV-1 test, Roche Molecular Systems, Basel, Switzerland) according to the manufacturer's protocol. The detection limit for this assay was 50 HIV-1-RNA copies/mL. Hepatitis C virus (HCV) RNA was determined using an available PCR procedure Kit (COBAS Amplicor, Roche Diagnosis, Barcelona, Spain) with a detection limit of 10 IU/mL.

Total DNA was extracted from peripheral blood mononuclear cells (PBMCs) using the MagNa Pure LC DNA isolation kit (Roche Diagnostics, S.L.U, Barcelona, Spain). HLA-B group alleles were genotyped using a reverse sequence-specific oligonucleotide bound to a fluorescently coded microsphere system (LABType SSO, RSSOIB, One Lambda, Canoga Park, CA), following the manufacturer's instructions.

### EXAMPLE 2. Characteristics of the studied subjects

Clinical and demographic characteristics of the subjects at baseline are shown in Table 2. No differences were observed in age, sex, transmission route, or HCV coinfection; in CD4+ or CD8+ T-cell counts; or in the CD4:CD8 ratio between the TC and PC. The TC group presented a shorter time since diagnosis than PC (3 [2-8] vs. 13 [10-17] years; p=0.005). No differences were found in HLA-B frequencies.

**Table 2. Patient' s characteristics**

| | **Transient Controllers, TC (n=8)** | **Persistent Controllers, PC (n=8)** | ***p*** |
|---|---|---|---|
| **Age (years)** | 41 [34-60] | 44 [40-46] | 0.635 |
| **Male sex, n (%)** | 5 (62.5) | 4 (50) | 0.614 |
| **Sexual transmission, n (%)** | 5 (62.5) | 4 (50) | 0.198 |
| **Time since diagnosis of HIV (years)** | 3 [2-8] | 13 [10-17] | **0.005** |
| **HCV RNA detected, n (%)** | 3 (37.5) | 3 (37.5) | 0.999 |
| **CD4+ T-cells (cell/µL)** | 676 [623-963] | 724 [609-985] | 0.817 |
| **CD8+ T-cells (cell/µL)** | 787 [553-1162] | 636 [432-1026] | 0.482 |
| **CD4:CD8 Ratio** | 0.86 [0.53-1.55] | 1.08 [0.93-1.47] | 0.406 |
| **HLA B57, n (%)** | 3 (37.5) | 1 (12.5) | 0.248 |
| **HLA B27, n (%)** | 1 (12.5) | 1 (12.5) | 0.999 |
| **HLA B35, n (%)** | 0(0) | 0(0) | 0.999 |

Values from TC are taken from -T2 and values from PC are taken from the first follow-up time point. Values are given as percentage for categorical variables or median and interquartile range for continuous variables. The Mann-Whitney U and Chi-squared tests were used. All p values <0.05 were considered significant and are highlighted in bold.

### EXAMPLE 3. A specific proteomic profile precedes the loss of natural HIV-1 control in Transient Controllers

Using a shotgun proteomic approach, a total of 293 proteins in plasma samples could be identified and quantified. To evaluate protein changes as an effect of time in the PC group, a PCA analysis and a Student's t-test was applied in paired samples in that group. No proteins significantly differed along time in PC (Figure 6). Due to this lack of change in proteins in the PC group, the following analysis was simplified to a single variable, which is expressed as the mean value of two consecutive longitudinal determinations.

To assess potential biomarkers for the loss of natural HIV-1 control, only preloss-of-control time points from TC were compared with PC. For all of the comparisons, a mean value of -T1 and -T2 was calculated.

As initial information regarding all of the quantified proteins, a partial least squares discriminant analysis (PLS-DA) applied before statistical analysis is shown in figure 7. The PLS-DA model demonstrated that there was a great difference between PC and TC, using the complete result quantified set of proteins that defined the model with an accuracy of 100%, which suggested that some of these quantified proteins may become a potential biomarker for the selected conditions. Eighteen proteins were found to exhibit statistically significant differences in plasma levels between TC before the loss of HIV-1 control and PC, within the complete set of important proteins defining the model (Table 1). As illustrated in the heatmap representation (Figure 2A), seven of them were downregulated and 11 were upregulated in TC compared with PC. These proteins also showed a reliable differentiation between the studied groups (Figure 2B). All together, these data indicate the feasibility of these proteins to be potential biomarkers for the loss of HIV-1 control.

STRING was used to analyze specific pathways and protein networks involving the differentially expressed proteins for biological interpretation. This tool determined two related inflammatory signaling pathways: leukocyte transendothelial migration and complement and coagulation cascade. The host proteins Galectin-3-binding protein (LG3BP) and Ficolin-2 (FCN2) were found to interact with gp120 (HIV function protein) and were upregulated (FC=2 and FC=1.59, respectively), whereas Intercellular adhesion molecule 1 (ICAM1), which interacts with tat protein, was downregulated in TC before the loss of natural HIV-1 control (FC=1.34).

To select and evaluate the performance of the potential biomarkers, we conducted a Random Forest analysis (Figure 3A), which elucidated that Coagulation factor XI, Alpha-1-antichymotrypsin, Ficolin-2, Galectin-3-binding protein and 14-3-3 protein zeta/delta were the main differentiators in a ranked list of the most significant proteins in order of their classification capability. It is important to highlight that these proteins were also the most significant variables in the univariate test.

A PCA analysis with these five proteins, and a good differentiation between both groups was observed, which corroborates their predictive strength of virological progression in EC (Figure 3B). Using logistic regression and ROC curves, (although all of them have a statistically significant area under the curve), only Galectin-3-binding protein could discriminate PC and TC patients with 100% sensitivity and specificity, which suggests that this protein could be the most reliable biomarker for the prediction of the spontaneous loss of control in EC (Figure 3C).

### EXAMPLE 4. Validation by Western blot

Western blot analyses were performed to verify differentially expressed proteins between PC and TC in the pre-loss-of-control-period and in searching for potential biomarkers. For this ascertainment, the five main protein differentiators mentioned before were selected (Figure 4A). The immunoblotting results confirmed the observations of the shotgun proteomic approach: Galectin-3 binding protein, Coagulation factor XI and 14-3-3 protein zeta/delta showed the highest differences between groups. Galectin-3-binding protein and 14-3-3 protein zeta/delta were upregulated in TC, whereas Coagulation factor XI was downregulated in this group of EC (Figure 4B). As suggested by the previous analyses, Galectin-3-binding was confirmed to be the main differentiator between groups.

### EXAMPLE 5. Protein changes in Transient Controllers as an effect of the loss of control

The protein changes in TC were also determined as an effect of detectable viremia. In this comparison, nine proteins showed statistically significant differences in plasma levels between TC pre-loss and post-loss time points (Table 3). The heatmap representation showed that 5 proteins were upregulated after the loss of control, whereas 4 proteins were downregulated after the loss of virological HIV-1 control (Figure 5A). Of note, SH3 domain-binding glutamic acid-rich like protein expression was almost 3-fold higher after the loss of control (Figure 5B).

**Table 3. List of the differentially plasma protein levels as a result of the loss of natural HIV-1 control in TC.**

| **Swiss-Prot Accessio n Number** | **Swiss-Prot Entry** | **Protein description** | **Gene name** | ***P* value** |
|---|---|---|---|---|
| P08603 | CFAH_HUMAN | Complement factor H | CFH | 4.56E-05 |
| P02765 | FETUA_HUMA N | Alpha-2-HS-glycoprotein | AHSG | 8.74E-03 |
| Q9H299 | SH3L3_HUMAN | SH3 domain-binding glutamic acid-rich-like protein 3 | SH3BGR L3 | 1.70E-02 |
| P06276 | CHLE_HUMAN | Cholinesterase | BCHE | 1.74E-02 |
| P35542 | SAA4_HUMAN | Serum amyloid A-4 protein | SAA4 | 1.86E-02 |
| P10909 | CLUS_HUMAN | Clusterin | CLU | 2.49E-02 |
| P00736 | C1R_HUMAN | Complement C1r subcomponent | C1R | 3.66E-02 |
| P25311 | ZA2G_HUMAN | Zinc-alpha- 2-glycoprotein | AZGP1 | 4.09E-02 |
| P02751 | FINC_HUMAN | Fibronectin | FN1 | 4.91E-02 |

Differences between groups were calculated using a Student's t test. Accession number is the MASCOT results of ESI-Ion trap searched from the SWISS-PROT database. FC; fold change.

## Claims

1. An *in vitro* method for determining the prognosis or progression of an HIV-infected subject having an undetectable viral load in the absence of antiretroviral treatment, which comprises:
(i) determining the expression level of at least one biomarker selected from the group consisting of Galectin-3-binding protein (LG3BP), α-1-antichymotrypsin (AACT), Ficolin-2 (FCN2), 14-3-3 protein zeta/delta (1433Z) and coagulation factor XI (FA11) in a sample from said subject, and
(ii) comparing the expression level of said at least one biomarker with a reference value,
wherein if the expression level of at least one biomarker selected from LG3BP, AACT, FCN2 and 1433Z is increased with respect to the reference value, and/or the level of expression of FA11 is decreased with respect to the reference value, then the subject shows bad prognosis, or
wherein if the expression level of at least one biomarker selected from LG3BP, AACT, FCN2 and 1433Z is decreased or lacks change with respect to the reference value, and/or the level of expression of FA11 is increased or lacks change with respect to the reference value, then the subject shows good prognosis.

2. The method according to claim 1, wherein the viral load is measured in a sample from a body fluid from said subject in the absence of an antiretroviral treatment for at least the last 6 months.

3. The method according to any one of claims 1 or 2, wherein the subject is classified as Elite Controller (EC).

4. The method according to any one of claims 1 to 3, wherein bad prognosis is **characterized by** an increase of the viral load over time above a detection limit in at least the last two body fluid samples extracted from the subject at different periods of time.

5. The method according to claim 4, wherein the last two body fluid samples extracted from the subject are extracted in an interval of at least 1 year.

6. The method according to any one of claims 1 to 5, wherein the detection limit of the viral load is 50 HIV RNA copies/ml of the body fluid sample in which the viral load is determined.

7. The method according to any one of claims 1 to 6, wherein the reference value is the expression level of the corresponding biomarker determined in a sample from one or more HIV-infected subjects that have not shown a viral load above the detection limit in at least the last 5 years in the absence of antiretroviral treatment.

8. The method according to any one of claims 1 to 7 wherein the sample in which the expression level of the at least one biomarker is determined is a blood sample.

9. The method according to any one of claims 1 to 8, wherein the determination of the expression level of the biomarker is carried out by determining the levels of the protein encoded by said gene.

10. The method according to any one of claims 1 to 9, comprising determining the level of expression of LG3BP, AACT, FCN2, 1433Z and FA11.

11. A kit comprising reagents adequate for the determination of the expression level of at least one biomarker selected from the group consisting of LG3BP, AACT, FCN2, 1433Z and FA11.

12. The kit according to claim 11, wherein said kit or assay device comprises reagents adequate for the determination of the expression level of at least two biomarkers selected from the group consisting of LG3BP, AACT, FCN2, 1433Z and FA11.

13. The kit according to any one of claims 11 or 12, wherein the biomarkers are proteins and the reagents adequate for the determination of the expression level are antibodies capable of specifically binding to said biomarker.

14. The kit according to any one of claims 11 to 13, wherein said reagents comprise at least 10% of the reagents present in the kit.

15. Use of a kit according to any one of claims 11 to 14 or of a reagent capable of determining the expression level of at least one biomarker selected from the group consisting of LG3BP, AACT, FCN2, 1433Z and FA11 for determining the prognosis or progression of an HIV-infected subject having an undetectable viral load in the absence of antiretroviral treatment.
